# EUROPEAN PATENT APPLICATION

(11) **EP 1 803 734 A1**
(43) Date of publication of application: **04.07.2007**
(21) Application number: 05790182.9
(22) Date of filing: 28.09.2005
(51) Int. Cl.: C07K 14/82, C12Q 1/68, G01N 27/62

(54) **NOVEL BONE METASTASIS MARKER PEPTIDE AND METHOD OF DIAGNOSING BONE METASTASIS BY USING THE SAME**

(30) Priority: 28.09.2004 JP 2004282822; 09.08.2005 JP 2005231193
(71) Applicant: KURUME UNIVERSITY, Kurume-shi, Fukuoka 830-0011 (JP)
(72) Inventor: TSURU, Michiyo, Kurume University School of Med., Kurume-shi, Fukuoka 830-0011 (JP); NAGATA, Kensei, Kurume University School of Med., Kurume-shi, Fukuoka 830-0011 (JP); SATA, Michio, Kurume University School of Med., Kurume-shi, Fukuoka 830-0011 (JP)
(74) Representative: Helbing, Jörg
(86) International application number: PCT/JP2005/018398
(87) International publication number: WO 2006/036002

(57) **Abstract**

It is intended to provide: a peptide which shows an increase in the expression amount in the serum of a cancer patient with the passage of time before the onset of bone metastasis and after the onset of bone metastasis, is adsorbed by a weak cation exchanger at pH 6, and has an apparent molecular weight determined by mass spectrometry of about 5,800; a peptide fragment comprising the amino acid sequence corresponding to from 10- to 62-positions of the amino acid sequence of matured azurocidin; and a method of diagnosing bone metastasis in a cancer patient **characterized by** comprising detecting the expression of one of the above peptides in a biological sample originating in the cancer patient. According to this method, bone metastasis can be found and treated at the early stage.

## Description

### Technical Field

The present invention relates to a peptide that can be used as a marker for bone metastasis, and a diagnosis method of bone metastasis characterized by determining the amount of the peptide in a test sample, and the like.

### Background Art

From 1981 to the present, cancer has been the biggest killer in Japan. In 2002, the number of deaths was 304,286, the death rate per 100 thousand population was 241.5, and the percentage to total deaths was 31.0%. It is estimated that, in 2015, 890,000 people will be affected with cancer in one year, ("GAN NO TOKEI (The Statistics for Cancer) '03" published by Foundation for Promotion of Cancer Research. Bone is the most common site of cancer metastasis next to lung and liver. Currently, bone metastasis is observed in 25-50% of the patients dying of cancer, and it is estimated that the number of the people who suffer from bone metastasis at the life's end will reach 70,000-150,000.

Cancer metastasis to bone, which causes bone fracture and neuroparalysis, is a serious problem in an advanced cancer. There are many bedridden patients who have symptoms due to bone metastasis but are not treated because they have an advanced cancer. It is the responsibility of medicine to maintain the high QOL of these patients and make their lives fulfilled.

For the diagnosis of cancer, some tumor markers in blood or urine are utilized in clinical practice. As bone metastasis markers, blood ICTP, blood or urine NTx, blood PICP (non-patent reference 1) and the like have been reported. In addition, patent reference 1 describes a diagnosis method of bone metastasis from a malignant tumor, comprising using PICP, PINP or BALP and osteocalcin as markers reflecting the osteoblast activity, and deoxypyridinoline and/or ICTP as marker(s) reflecting the osteoclast activity.

However, at home and abroad, no marker permitting clinical application as a specific marker for human bone metastasis has been discovered. As the situation stands, the diagnosis of bone metastasis is currently made by a diagnostic imaging such as bone scintigraphy, positron-CT (PET), MRI, CT and the like or biopsy, which makes early detection and early treatment impossible to result in marked impairment of QOL of the patients.
[patent reference 1] WO 00/11480
[non-patent reference 1] Miura H., Yamamoto I., Takada M., Kigami Y., Ohta T., Yuu I., Hamanaka Y., Matsushita R. and Morita R. "Diagnostic validity of bone metabolic markers for bone metastasis" Endocr. J., 44(5): 751-7 (1997)

### Disclosure of the Invention

It is therefore an object of the present invention to provide a marker specific for human bone metastasis enabling early detection of bone metastasis of cancer, and a method for an early diagnosis of bone metastasis, which comprises determining the amount of the marker in a biological sample from a cancer patient.

To accomplish the above-mentioned object, the present inventors analyzed the proteome in serum samples obtained from cancer patients before and after the onset of bone metastasis by time series by a mass spectrometry using a protein chip, which has made a significant contribution to the search of disease markers including tumor markers. As a result, they found a peptide with a molecular weight of about 5,800, which increases in serum over time from before the onset of bone metastasis to the beginning of a treatment after the onset thereof, and decreases by the treatment. The present inventors further analyzed the peptide by various proteome analysis methods using a mass spectrometry, and found that the peptide is a fragment of a neutrophil-derived heparin-binding protein azurocidin (also referred to as Cationic Antimicrobial Protein (CAP) 37), which is known as a monocyte chemoattractant, which resulted in the completion of the present invention.

Accordingly the present invention provides:
[1] a peptide having the following properties:
   (a) increased expression in the serum of a cancer patient over time from before the onset of bone metastasis to after the onset thereof;
   (b) adsorbing to a weak cation exchanger under the condition of pH 6;
   (c) apparent molecular weight by mass analysis: about 5,800,
[2] a peptide consisting of the same or substantially the same amino acid sequence as the amino acid sequence of amino acid numbers 10-62 of the amino acid sequence shown in SEQ ID NO:2,
[3] an antibody against the peptide of the above-mentioned [1] or [2],
[4] a diagnosis method of bone metastasis in a cancer patient, which comprises detecting the expression of the peptide of the above-mentioned [1] or [2] in a biological sample derived from the patient,
[5] the method of the above-mentioned [4], wherein the biological sample is plasma or serum,
[6] the method of the above-mentioned [4] or [5], which comprises bringing the biological sample into contact with a cation exchanger, subjecting the component adsorbed to the cation exchanger in the sample to a mass spectrometry, and detecting a peptide having the molecular weight of about 5,800,
[7] the method of the above-mentioned [4] or [5], which comprises detecting the peptide of the above-mentioned [1] or [2] using the antibody of the above-mentioned [3], and
[8] the method of any of the above-mentioned [4] - [7], which comprises obtaining a biological sample from a cancer patient in time series, and assaying the time course expression of the peptide of the above-mentioned [1] or [2] in the sample.

Since the serum level of the peptide marker for bone metastasis of the present invention begins to increase from a time point considerably earlier than the exteriorization of the symptoms of bone metastasis, the marker makes it possible to detect and prevent bone metastasis earlier than by the diagnostic imaging currently utilized. The peptide marker has a further advantage that it enables an unambiguous diagnosis because its targets are limited. Furthermore, since the analysis can be performed with a small amount of patient serum, the burden on the patient is less.

### Brief Description of the Drawings

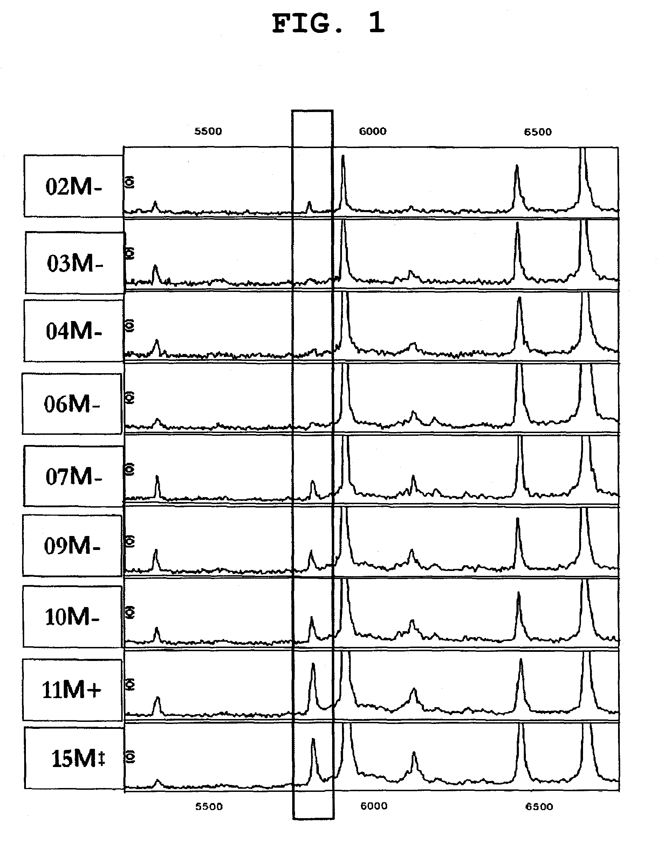

Fig. 1 shows the mass spectra of the component trapped to a cation exchanger on a cation exchange protein chip, wherein the chip was contacted with serum samples obtained in time series from a liver cancer patient. The horizontal axis shows molecular weight. The number in the left side of the chart shows the number of months from consultation of the patient at the time of serum sampling, (-) shows before the onset of bone metastasis, (+) shows after the onset of bone metastasis, and (‡) shows after bone metastasis and after the beginning of treatment, respectively.

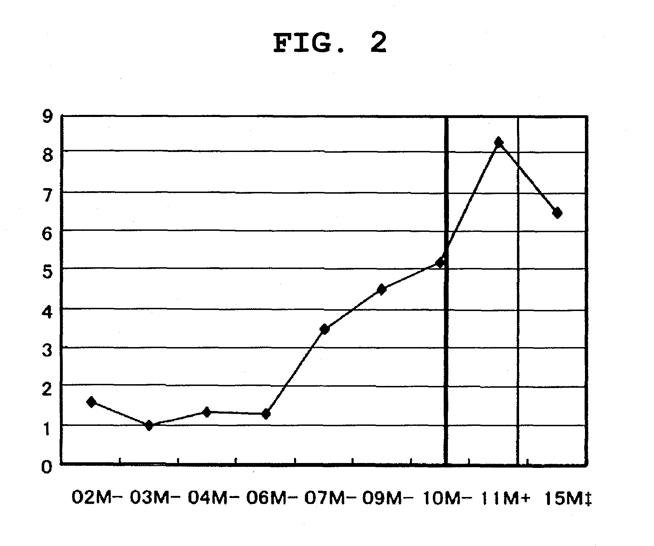

Fig. 2 shows a time course of the average peak intensity of the peak that appeared at the molecular weight of 5,813 in Fig. 1. The number in the horizontal axis shows the number of months from consultation of the patient at the time of serum sampling, (-) shows before the onset of bone metastasis, (+) shows after the onset of bone metastasis, and (‡) shows after bone metastasis and after the beginning of treatment, respectively.

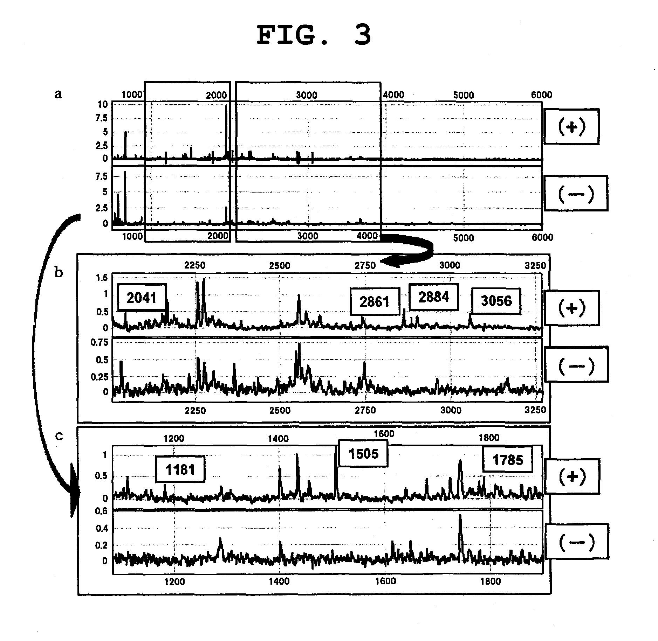

Fig. 3 shows the results of mass spectrometry analyses using SELDI of the elution fractions (with 1 M NaCl) from the cation exchanger column (pH 6.0) of serum (+) in which the bone metastasis marker with molecular weight of 5,813 was detected by cation exchange chip analysis and serum (-) in which the marker was not detected, following on chip reduction and on chip trypsin digestion. (a) shows the whole chart, (b) shows close up of M/Z 2,000-3,250, and (c) shows close up of M/Z 1,000-2,000, respectively. In each chart, the upper panel shows (+) serum, the bottom panel shows (-) serum. The number in box shows mass (M/Z) of a (+) serum-specific peak.

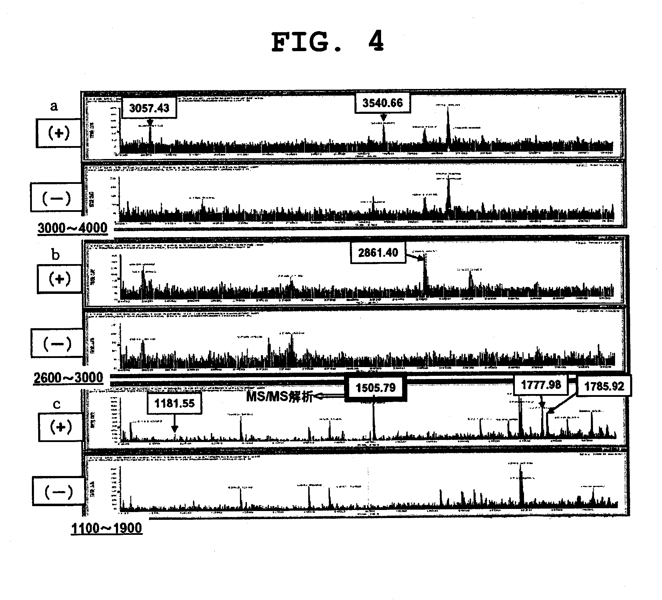

Fig. 4 shows the results of mass spectrometry analyses using PCI-Qstar of the elution fractions (with 1 M NaCl) from the cation exchanger column (pH 6.0) of serum (+) in which the bone metastasis marker with molecular weight of 5,813 was detected by cation exchange chip analysis and serum (-) in which the marker was not detected, following on chip reduction and on chip trypsin digestion. (a) shows the whole chart, (b) shows close up of M/Z 3,000-4,000, and (c) shows close up of M/Z 2,600-3,000, respectively. In each chart, the upper panel shows (+) serum, the bottom panel shows (-) serum. The number in box shows mass (M/Z) of a (+) serum-specific peak.

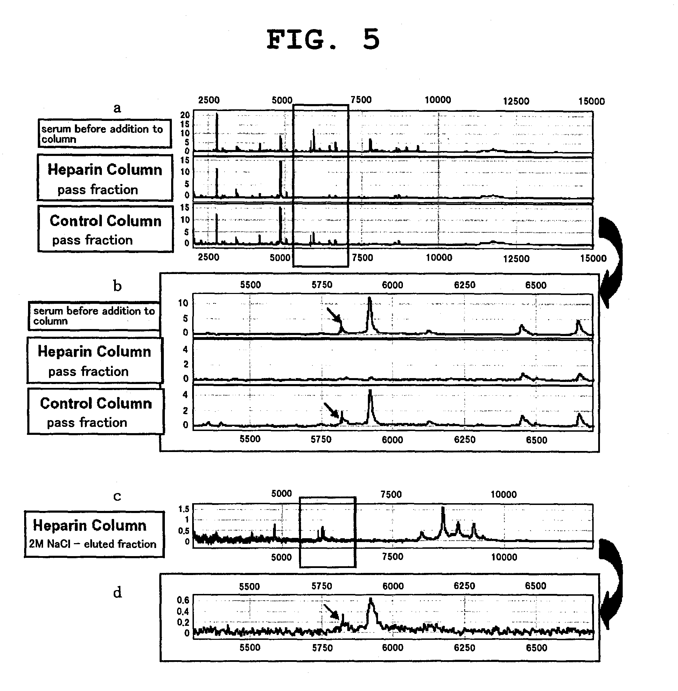

Fig. 5 shows the results of mass spectrometry analyses of the sample before application to heparin column (upper panel), the fraction not adsorbed to (passing trough) the column (middle panel) and the fraction passing trough a control column (IDM Affinity Beads:C540-0019) (a and b), and the fraction eluted from the heparin column with 2 N NaCl (c and d) of serum in which the bone metastasis marker with molecular weight of 5,813 was detected by cation exchange chip analysis. (a) and (c) show the whole charts, and (b) and (d) shows close ups of M/Z 5,250-6,750. The arrow shows the object peak.

### Best Mode for Embodying the Invention

The present invention provides a peptide useful as a bone metastasis marker in human. The peptide (hereinafter also referred to as "the peptide of the present invention") is characterized by the following properties:
(a) increased expression in the serum of a cancer patient over time from before the onset of bone metastasis to after the onset thereof;
(b) adsorbing to a weak cation exchanger under the condition of pH 6;
(c) apparent molecular weight by mass analysis: about 5,800.

Herein "the onset of bone metastasis" means to exhibit symptoms of bone metastasis (e.g., pain of, numbness in and paralysis of bone and limbs, bone fracture etc.). "Before the onset of bone metastasis" includes both a state where the bone metastasis has occurred but the patient is asymptomatic, and before bone metastasis actually occurs. "After the onset of bone metastasis" means after the patient begins to exhibit symptoms of bone metastasis, and before the remedy of the symptoms is observed by a treatment for bone metastasis. While there is an interindividual difference in the time period when the serum level of the peptide begins to elevate, it is, for example, about 4 to about 12 months before the onset.

The peptide of the present invention is characterized by adsorbing to a weak cation exchanger, in particular, the cation exchanger immobilized on the cation exchange protein chip CM10 or WCX2 ProteinChip Array (model number: C553-0075 or C553-0026) manufactured by Ciphergen Biosystems, at least under the conditions of pH 6. More specifically, the peptide of the present invention adsorbs to a weak cation exchanger at a level detectable by a mass spectrometry when the pH is 8.5 or less, and more efficiently adsorbs at pH 6.5 or less. When the peptide is detected by a mass spectrometry, however, the particularly preferable pH condition is pH 6 in view of less noise peak adsorption. The apparent molecular weight of the peptide by mass spectrometry is about 5,800. In particular, the peptide is characterized by generating a peak at the position of molecular weight of 5,813±0.5% (preferably +0.3%, more preferably ±0.1%) when analyzed by mass spectrometry according to the MALDI method (matrix: sinapic acid (SPA)) using the above-mentioned chip.

Alternatively, the peptide of the present invention is defined by consisting of the same or substantially the same amino acid sequence as the amino acid sequence of amino acid numbers 10-62 of the amino acid sequence shown in SEQ ID NO:2. The polypeptide consisting of the amino acid sequence shown in SEQ ID NO: 2 is a precursor (prepro protein) of human azurocidin, which is a neutrophil-derived heparin-binding protein known as a monocyte chemoattractant. The sequence consisting of 26 amino acids of the amino acid numbers -26 to -1 is putative signal peptide, and it is predicted the 3 amino acid residues at C-terminus are further cleaved to give the mature protein. However, the existence of the proteolytic product of azurocidin consisting of the amino acid sequence of the amino acid numbers 10-62 (also referred to as "AZU(36-88)" because the sequence corresponds to the amino acid sequence from position 36 to position 88 of the prepro protein) has never been known, and the product is a novel peptide.

The "substantially the same amino acid sequence" as AZU(36-88) means the amino acid sequence of the part corresponding to AZU(36-88) in a variant or polymorphism of azurocidin protein containing substitution, deletion or insertion of amino acid(s) wherein the substitution, deletion or insertion is present in the amino acid sequence of the amino acid numbers 10-62 of the amino acid sequence shown in SEQ ID NO:2.

The present invention also provides a method for the diagnosis of bone metastasis in a cancer patient by using the peptide of the present invention as a bone metastasis marker, and detecting the expression in the patient. Herein "the diagnosis of bone metastasis" is used to mean not only determination of whether or not bone metastasis has already occurred, but also determination of whether or not the possibility of bone metastasis in the near future is high.

The cancer patient who can be the subject for the diagnosis method of the present invention is not particularly limited by the primary lesion of cancer. The method is applicable to the human affected with any cancer capable of causing bone metastasis (e.g., breast cancer, lung cancer, prostate cancer, thyroid cancer, kidney cancer, gastric cancer, liver cancer, uterine cancer, colorectal cancer and the like), and preferably applied to patients with breast cancer, lung cancer and prostate cancer, because these cancer patients especially have high percentages of bone metastasis. The age and sex of the patient and the like are not limited at all.

While the biological sample to be the test sample derived from the patient is not particularly limited, less-invasively obtainable one is preferable and, for example, a secretion product from human such as blood, serum, plasma, saliva, mucous membrane, tear or the like, or one obtained by biopsy is used.

When serum or plasma is used, it can be prepared by drawing blood from a patient according to a conventional method and separating a liquid component therefrom. Since the molecular weight of the peptide of the present invention, the detection target, is about 5,800, a high molecular weight protein fraction and the like may be previously removed using a spin column as necessary. As mentioned below, when the detection target is the mRNA encoding the peptide of the present invention, a total RNA or poly(A)+ RNA fraction may be extracted and purified from the obtained blood using a conventional method.

The expression of the peptide of the present invention in the biological sample can be assayed by detecting the peptide or the mRNA encoding the same using a conventionally known method. For example, when detecting the peptide of the present invention, the detection can be performed by bringing the sample into contact with a cation exchanger followed by washing under suitable conditions, and detecting a peptide with molecular weight of about 5,800 from the component bound to the cation exchanger. The "cation exchanger" herein means an ion exchanger that has negatively charged groups and traps cations, and classified into a strong cation exchanger (e.g., an insoluble carrier having a styrene or acrylic matrix structure with a functional group such as propylsulfonic acid, benzenesulfonic acid, etc., and the like), and a weak cation exchanger (e.g., an insoluble carrier having a styrene or acrylic matrix structure with a functional group such as carboxymethyl group, carboxyethyl group, etc., and the like). Any of the exchangers can be used without limitation in the present inventive method, but a weak cation exchanger is preferably used.

The washing can be performed with water or buffer. For example, pH used is set according to the isoelectric point of the target marker, therefore Tris-HCl buffer, phosphate buffer, borate buffer, acetate buffer or the like at pH 4-8 is used.

The detection of a peptide with molecular weight of about 5,800 from the component bound to the cation exchanger can be performed, for example, by adding an eluent to the cation exchanger changing its salt concentration (e.g., 100-1000 mM aqueous sodium chloride solution and the like) to elute the adsorbed component, followed by concentration as necessary, and subjecting the eluate to various determination methods of molecular weight such as gel electrophoresis, methods combining various separation and purification methods (e.g., ion exchange chromatography, hydrophobic chromatography, affinity chromatography, reversed-phase chromatography and the like), ionization methods (e.g., electron-impact ionization method, field desorption method, secondary ionization method, high-speed atom collision method, matrix-assisted laser desorption/ionization (MALDI) method, electrospray method and the like) and mass spectrometer (e.g., double-focusing mass spectrometer, quadrupole spectrometer, time-of-flight mass spectrometer, Fourier-transform mass spectrometer, ion cyclotron mass spectrometer and the like), and the like.

The "molecular weight of about 5,800" is the wording taking the range of error with each determination method into account. For example, in the case of a method using a mass spectrometer, it is preferable to measure the peak strength of a peak appearing at the position of molecular weight of 5,813±0.5% (preferably ±0.3%, more preferably ±0.1%).

In the detection of the peptide with molecular weight of about 5,800 in the diagnosis method of the present invention, as the most preferable determination methods, a method combining cation exchange chromatography and time-of-flight mass spectrometry (TOF-MS) to collectively determine the masses of all of the biological sample components trapped on the cation exchanger under certain conditions, especially a method comprising immobilizing the cation exchanger onto the surface of the probe used for time-of-flight mass spectrometry, bringing the probe surface into contact with a test sample followed by washing under suitable conditions, and determining the masses the components trapped on the probe surface with a time-of-flight mass spectrometer, and the like can be mentioned. The same cation exchanger as mentioned above can be used, and a weak cation exchanger is particularly preferable. A probe compatible to time-of-flight mass spectrometer wherein a weak cation exchanger is immobilized onto the surface thereof includes, but not limited to, the aforementioned CM10 or WCX2 Protein Chip Array (model number: C553-0075 or C553-0026) manufactured by Ciphergen Biosystems, and the like. The washing can be performed using the above-mentioned buffer and the like, at room temperature and the like. The mass spectrometry is preferably performed by MALDI method using a suitable matrix (MALDI-TOFMS). As matrix molecule used, for example, sinapic acid (SPA), saturated 2,5-dihydroxybenzoic acid (DHB), indoleacrylic acid (IAA), cinnamic acid and the like can be mentioned.

The peptide of the present invention in the diagnosis method of the present invention can also be detected using an antibody against the peptide. The antibody against the peptide of the present invention can be prepared, for example, by isolating and purifying the peptide of the present invention from a biological sample derived from a cancer patient expressing the same, and immunizing an animal with the peptide as an antigen. Alternatively, when only the small amount of the peptide is obtained and the like, a large amount of the peptide of the present invention can be prepared by partially digesting the peptide with a peptidase and the like, determining the amino acid sequence of the obtained fragment by Edman method and the like, synthesizing oligonucleotides hybridizable to a nucleic acid encoding the peptide on the basis of the sequence, obtaining cDNA encoding the peptide by hybridization method, using the oligonucleotides as probes and cDNA library derived from a patient as template, or obtaining cDNA encoding the peptide by RT-PCR using the oligonucleotides as primers and the patient-derived RNA as template, incorporating the cDNA into a suitable expression vector and introducing the same into a suitable host cell, culturing the obtained transformant and recovering the recombinant peptide. Alternatively, the peptide of the present invention can be obtained using *in vitro* transcription and translation systems with the cDNA obtained as mentioned above.

Alternatively, by using a tandem mass spectrometry (MS/MS) in the above-mentioned detection method combining a cation exchanger and mass spectrometry, the amino acid sequence of the peptide of the present invention can be directly identified. Based on the sequence information, whole or a part of the peptide can be synthesized and utilized as the antigen (hapten). As a method for identifying peptide using MS/MS method, *de novo* sequencing method comprising analyzing MS/MS spectrum to determine the amino acid sequence, and a method comprising searching a database using sequence information (mass tag) contained in MS/MS spectrum to identify the peptide, and the like can be mentioned.

As mentioned above, the peptide of the present invention is a fragment of human azurosidin, whose amino acid sequence is the same or substantially the same sequences as the amino acid sequence (AZU(36-88)) of the amino acid numbers 10-62 of the amino acid sequence shown in SEQ ID NO: 2. Accordingly, the antibody against the peptide of the present invention are preferably prepared, for example, by synthesizing whole or a part of the sequence of AZU(36-88) based on the above-mentioned amino acid sequence information using a known peptide synthesis method or cleaving an isolated azurocidin with a suitable peptidase and the like to give a peptide fragment containing whole or a part of the sequence of AZU(36-88), and using the same as the immunogen.

The antibody against the peptide of the present invention (hereinafter also referred to as "the antibody of the present invention") may be any of a polyclonal antibody and a monoclonal antibody, and produced by well known immunological techniques. In addition, the antibody includes not only a complete antibody molecule but also a fragment thereof, for example, Fab, F(ab')₂, ScFv, minibody and the like can be mentioned.

For example, a polyclonal antibody can be obtained by giving the peptide of the present invention or a partial peptide thereof [may be prepared as a complex cross-linked with a carrier protein such as bovine serum albumin or KLH (Keyhole Limpet Hemocyanin), if necessary] as the antigen, along with a commercially available adjuvant (e.g., Freund's complete or incomplete adjuvant), to an animal by subcutaneous or intraperitoneal administration about 2 to 4 times at intervals of 2 to 3 weeks (the antibody titer of serum separated from drawn blood determined by a commonly known antigen-antibody reaction, and its elevation confirmed in advance), collecting whole blood about 3 to about 10 days after final immunization, and purifying the antiserum. Animals to be administered with the antigen include mammals such as rat, mouse, rabbit, goat, guinea pig and hamster.

A monoclonal antibody can also be prepared by a cell fusion method (e.g., Takeshi Watanabe, saibouyugouhou no genri to monokuronaru kotai no sakusei, Akira Taniuchi and Toshitada Takahashi, eds., "monokuronaru kotai to gan-kiso to rinsho-", pp. 2-14, Science Forum Publishing, 1985). For example, the peptide of the present invention or a partial peptide thereof is given to a mouse, along with a commercially available adjuvant, 2 to 4 times by subcutaneous or intraperitoneal administration, its spleen or lymph node is collected about 3 days after final administration, and leukocytes are separated. These leukocytes are fused with myeloma cells (e.g., NS-1, P3X63Ag8, etc.) to yield a hybridoma that produces a monoclonal antibody against the peptide. The cell fusion may be achieved by the PEG method [J. Immunol. Methods, 81(2): 223-228 (1985)] or the voltage pulsation method [Hybridoma, 7(6): 627-633 (1988)]. A hybridoma that produces the desired monoclonal antibody can be selected by detecting in the culture supernatant an antibody that specifically binds to an antigen using well-known EIA, RIA, or the like. Cultivation of a hybridoma that produces a monoclonal antibody can be conducted *in vitro,* or *in vivo* in mice or rats, preferably in ascites fluid of mouse, and the resulting antibody can be obtained from a hybridoma culture supernatant or animal ascites fluid, respectively.

The diagnosis method of the present invention using the antibody of the present invention is not particularly limited, and any method of measurement can be used, as long as it is a measurement method wherein the amount of antibody, antigen or antibody-antigen complex corresponding to the amount of antigen in the test sample is detected by a chemical or physical means and is applied to a standard curve generated using standard solutions containing known amounts of antigen. For example, nephelometry, the competitive method, the immunometric method and the sandwich method are preferably used.

As examples of the labeling agent used for the assay using a labeled substance, a radioisotope, an enzyme, a fluorescent substance, a luminescent substance and the like can be used. As examples of the radioisotope, [¹²⁵I], [¹³¹I], [³H], [¹⁴C] and the like can be used. As the enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-avidin system can also be used for binding of an antibody or an antigen and a labeling agent.

In insolubilizing the antigen or antibody, physical adsorption may be used, and a chemical bond in common use to insolubilize or immobilize a protein or an enzyme or the like, may also be used. As the carrier, insoluble polysaccharides such as agarose, dextran and cellulose, synthetic resins such as polystyrene, polyacrylamide and silicone, glass and the like can be mentioned.

In the sandwich method, the amount of the peptide of the present invention in a test sample can be quantified by reacting the test sample to an antibody of the present invention insolubilized (primary reaction) and further reacting to another antibody of the present invention labeled (secondary reaction), and thereafter measuring the amount (activity) of the labeling agent on the insolubilizing carrier. The primary reaction and the secondary reaction may be conducted in the reverse order, and may be conducted simultaneously or after a time lag.

The antibody of the present invention can be used for a measurement system other than the sandwich method, for example, the competitive method, the immunometric method or nephelometry and the like.

In the competitive method, the antigen and the labeled antigen in the test sample are competitively reacted with the antibody, after which the unreacted labeled antigen (F) and the antibody-bound labeled antigen (B) are separated (B/F separation), the amount labeled of either B or F is measured, and the amount of antigen in the test sample is quantified. For this reaction method, the liquid phase method, wherein a soluble antibody is used as the antibody and B/F separation is conducted using polyethylene glycol, a second antibody against the above-described antibody (first antibody), and the like, and the solid phase immobilization method, wherein a solid-phase-immobilized antibody is used as the first antibody or the first antibody used is a soluble one and a solid-phase-immobilized antibody is used as the second antibody, can be used.

In the immunometric method, an antigen in a test sample and an immobilized antigen are competitively reacted with a given amount of labeled antibody, and thereafter the solid phase is separated from the liquid phase, or an antigen in a test sample is reacted with an excess amount of labeled antibody, an immobilized antigen is then added to bind the unreacted labeled antibody to the solid phase, and the solid phase is separated from the liquid phase. Subsequently, the amount of labeled antibody in either phase is measured to quantify the amount of antigen in the test sample.

Also, in nephelometry, the amount of insoluble precipitate resulting from an antigen-antibody reaction in the gel or in the solution is measured. Even when the amount of antigen in the test sample is small and only a small amount of precipitate is obtained, laser nephelometry, which utilizes laser scattering, and the like are preferably used.

In applying these individual immunological measurement methods to the quantitation method of the present invention, it is unnecessary to set special conditions, procedures and the like. Making ordinary technical considerations for those skilled in the art to the ordinary conditions and procedures in each method, a measurement system for the peptide of the present invention can be constructed. For details of these general technical means, compendia, books and the like can be referred to.

For example, edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like can be referred to.

Alternatively, as another diagnosis method of the present invention using the antibody of the present invention, a method comprising immobilizing the antibody on the surface of the above-mentioned probe fit with a mass spectrometer, bringing a test sample into contact with the antibody on the probe, subjecting the biological sample component trapped by the antibody to mass spectrometry, and detecting a peptide with molecular weight of about 5,800 can be mentioned.

In the diagnosis method of the present invention, when the expression of the peptide of the present invention in a biological sample is assayed by detecting mRNA encoding the peptide (namely, mRNA encoding azurocidin producing the peptide by its degradation, specifically, RNA comprising the base sequence shown in SEQ ID NO:1), the assay can be performed by Northern hybridization, RT-PCR and the like using a nucleic acid (probe) capable of hybridizing to the mRNA under stringent conditions, or a set of oligonucleotides capable of functioning as primers to amplify whole or a part of the mRNA. The nucleic acid to be used as a probe may be a DNA or RNA, or a DNA/RNA chimera. Preferably, DNA can be mentioned. The nucleic acid may be a double strand or a single strand. In the case of a double-strand polynucleotide, it may be a double-strand DNA, a double-strand RNA or a DNA:RNA hybrid. The length of the nucleic acid is not particularly limited as long as it can specifically hybridize to a target mRNA and, for example, it is not less than about 15 bases, preferably not less than about 30 bases. The nucleic acid is preferably labeled with a label to enable detection or quantitation of a target mRNA. As examples of the labeling agent, a radioisotope, an enzyme, a fluorescent substance, a luminescent substance and the like can be used. As examples of the radioisotope, [³²P], [³H], [¹⁴C] and the like can be used. As the enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-(strepto)avidin system can also be used for binding of a probe and a labeling agent.

An oligonucleotide set to be used as a primer is not particularly limited as long as it can specifically hybridize to each of a base sequence (sense strand) of mRNA encoding azurosidin and a base sequence complementary thereto (antisense strand), and amplify a DNA fragment sandwiched therebetween and, for example, a set of oligo DNAs each designed to have a length of about 15 to about 100 bases, preferably about 15 to about 50 bases, and amplify about 100 bp to several kbp DNA fragments can be mentioned.

For quantitative analysis of gene expression of the peptide of the present invention using a trace amount of an RNA sample, competitive RT-PCR or real-time RT-PCR is preferably used. Competitive RT-PCR is a method comprising calculating the amount of an object DNA by carrying out a competitive amplification reaction in a reaction mixture in the co-presence of a known amount of other template nucleic acid as a competitor, which can be amplified by a primer set capable of amplifying the object DNA, and comparing the amounts of the amplification products. When using competitive RT-PCR, therefore, the reagent of the present invention can further contain, besides the above-mentioned primer set, a nucleic acid which is amplified by the primer set to produce an amplification product distinguishable from the object DNA (e.g., amplification product different from the object DNA in size, amplification product showing different migration pattern by a restriction enzyme treatment and the like). This competitor nucleic acid may be a DNA or an RNA. In the case of a DNA, cDNA is synthesized from an RNA sample by a reverse transcription reaction and a competitor is added to perform PCR, and in the case of an RNA, it may be added to an RNA sample from the start to perform RT-PCR. In the latter case, an absolute amount of the original mRNA can also be assumed since the efficiency of the reverse transcription reaction is taken into consideration.

On the other hand, real-time RT-PCR does not require electrophoresis, since the amplification amount by PCR can be monitored real-time, and the gene expression of the peptide of the present invention can be analyzed more rapidly. Generally, monitoring is performed using various fluorescent reagents. These include reagents (intercalator) emitting fluorescence by binding to double stranded DNA such as SYBR Green I, ethidium bromide and the like, nucleic acids usable as the above-mentioned probes (the nucleic acid hybridizes to the target nucleic acid within amplification region), wherein the both ends are respectively modified with a fluorescent substance (e.g., FAM, HEX, TET, FITC etc.) and a quenching substance (e.g., TAMRA, DABCYL etc.) and the like.

The nucleic acid to be used as the above-mentioned probe can be cDNA encoding azurocidin or a DNA encoding a fragment thereof (e.g., AZU(36-88) etc.), or can also be obtained by chemically synthesizing a part or whole of the base sequence and/or its complementary strand sequence based on the information of the base sequences (see SEQ ID NO:1) can also be obtained by chemically synthesizing, using a commercially available DNA/RNA automatic synthesizer and the like. The aforementioned oligonucleotide set to be used as a primer can also be obtained by chemically synthesizing a part or whole of the base sequence and/or its complementary strand sequence based on the aforementioned information of the base sequences.

The diagnosis method of the present invention is preferably performed by obtaining a biological sample from a cancer patient in time series, and examining the time course of the expression of the peptide of the present invention in the sample. The sampling interval of the biological sample is not particularly limited, given the early detection of bone metastasis, the object of the present invention, it is desirable to sample as frequent as possible within a scope which does not compromise the patient's QOL. For example, when a blood (serum, plasma) is used as the sample, the blood sample was preferably collected about one month interval. The expression of the peptide of the present invention in each sample is examined, when the expression of the peptide increased over time, it is possible to determine that bone metastasis has occurred or there is a high possibility that bone metastasis will occur in the near future.

As shown in the below-mentioned Examples, the expression the peptide of the present invention shows a tendency to decrease when the treatment against bone metastasis was successful after the onset of bone metastasis. Accordingly, the diagnosis method of the present invention can also be used to an early determination of the effect of the treatment in a patient after the onset of bone metastasis. For example, when the expression of the peptide of the present invention did not decrease or continued to increase even a certain period passed from the beginning of the treatment against bone metastasis, it is possible to determine that the change of the treatment strategy and the like should be considered. Also, when the increase of the expression of the peptide of the present invention was observed before bone metastasis actually occurred, and a prophylactic treatment against bone metastasis was carried out, it can be determined whether or not the prophylactic effect is obtained by monitoring the expression of the peptide of the present invention.

The present invention is explained in more detail in the following by referring to Examples, it goes without saying that they do not limit the scope of the present invention in any way. The measurement was performed using the Protein Chip system of Ciphergen Biosystems Inc. and the reagents attached thereto, if not otherwise specified.

### Example 1 Detection of bone metastasis marker

From a liver cancer patient who consulted the Second Internal Medicine, Kurume University School of Medicine, blood samples were collected in time series from before the onset of bone metastasis (2 months after consultation) to after the onset of bone metastasis (11 months thereafter) and after the beginning of the treatment for bone metastasis (15 months thereafter), and sera were prepared according to a conventional method. To 1 µL of serum was added 9 µL of denaturation buffer and the mixture was thoroughly mixed. The mixture was incubated on ice for 10 min, and 90 µL of protein chip experiment buffer (pH 6) was added.

150 µL of protein chip experiment buffer was added to cation exchange protein chips (CM10; model number: C5730075), and the chips were equilibrated at room temperature for 5 min with shaking. These operations were repeated twice. The above-mentioned prepared serum samples (100 µL each) were added onto the protein chips, and incubated at room temperature for 30 min with shaking. 150 µL of protein chip experiment buffer (pH 6) was added to the protein chips and the chips were equilibrated at room temperature for 5 min with shaking. These operations were repeated three times. 200 µL of MilliQ water was added to each protein chip, the chip was rinsed and desalted. These operations were repeated twice. The protein chips were air-dried, 0.5 µL of saturated energy absorption molecule (sinapic acid) solution was added and the chips were air-dried. These operations were repeated twice. Thereafter, the determination was performed with a protein chip reader.

Baseline correction, molecular weight calibration and normalization treatment were carried out. For the analysis of a single marker, t-test, ROC analysis, and multimarker analysis (principal component analysis, clustering, classification analysis) were performed.

The results are shown in Figs. 1 and 2. The peak intensity at the position of molecular weight of 5,813 increased over time from 7 months after consultation before the onset of bone metastasis (until 11 months after consultation), and decreased after beginning of the treatment for bone metastasis (15 months after consultation).

### Example 2 Purification of bone metastasis marker

### (1) Consideration of purification conditions

Mass spectrometry was performed for the serum samples wherein a peak was detected at the position of molecular weight of 5,813 in the same manner as in Example 1 using a cation exchange protein chip (CM10; model number: C5730075) or an anion exchange protein chip (Q10; model number: C5730080), changing the pH of the protein chip experiment buffer (50 mM phosphate buffer) within the range of 5.0-8.5. As a result, no peak was detected at the position of molecular weight of 5,813 at any pH when the anion exchange protein chip was used. On the other hand, when the cation exchange protein chip was used, the objective peak was efficiently detected at pH 6.5 or less, and detected even at pH 8.5 although the detection sensitivity was low. These results suggest the possibility that the isoelectric point (pI) of the peptide corresponding to the objective peak is higher than pH 8.5. Since the (noise) adsorption of peaks other than the objective peak is few, thereafter pH 6.0 was adopted as the standard adsorption condition.

Next, the salt concentration to elute the peak at the position of molecular weight of 5,813 adsorbed to the cation exchange protein chip under the adsorption condition of pH 6.0 was examined. After treating with 0, 100, 200, 300, 500 mM and 1 M NaCl, respectively, the protein chip was subjected to mass spectrometry. As a result, the loss of the objective peak was confirmed at 200 mM NaCl.

### (2) Purification of marker peptide

To serum wherein a peak was detected at the position of molecular weight of 5,813 ((+)serum) or serum wherein the peak was detected ((-)serum) (500 µl each) was added 4.5 ml of denaturation buffer (urea/thiourea/CHAPS) and thoroughly mixed. The mixture was incubated on ice for 10 min, 5-fold diluted with 50 mM phosphate buffer (pH 6) and centrifuged at 15,000 rpm for 10 min. The supernatant was applied to a cation exchange column (CM Ceramic Hyper D F Spin Columus: C540-0024) equilibrated with 50 mM phosphate buffer (pH 6) (1000 µl), and stepwise elution was carried out with 100, 200, 300, 500 mM, 1 M and 1 M NaCl. Each elution fraction (5 µl) was added onto a normal phase protein chip (NP20), the chip was washed with MilliQ water, 100% sinapic acid was added thereto as a matrix and mass spectrometry was performed by MALDI method. As a result, in (+)serum, the objective peak began to elute in a small amount at 300 mM NaCl, and was eluted in maximum at 1 M NaCl. The elution fraction of each serum sample at 1 M NaCl was recovered, and centrifuged at 15,000 rpm for 10 min using an ultrafiltration membrane (5 kDa cutoff; VIVA SPIN). The operations comprising addition of 50 mM phosphate buffer (pH 6) (500 µl) and centrifugation at 15,000 rpm for 10 min were repeated twice to concentrate and desalt the sample (final volume 30 µl, peptide concentration 0.2 mg/ml). The sample after concentration and desalting was subjected to mass spectrometry in the same manner as mentioned above. As a result, in the concentrated sample of (+)serum, the objective peak was efficiently detected.

### Example 3 Identification of bone metastasis marker

### (1) On Chip reduction

The concentrated and desalted sample of (+)serum obtained in Example 2(2) was added onto NP20 chip at 0.5 µl per spot, and the chip was washed twice with MilliQ water (5 µl). After the addition of 5 mM DTT (in 25 mM NH₄HCO₃, pH 8) (5 µl), the chip was subjected to reduction treatment at 70°C and dried. The saturated sinapic acid (0.5 µl) added twice and mass spectrometry was performed. As a result, mass increase of about 2 Da was observed compared with before the reduction (5810.6+H), which suggests that the objective peptide contains an S-S bond. Thus, a reduction treatment was to be performed prior to the following trypsin digestion.

### (2) Peptide mass fingerprinting

Each of the concentrated and desalted samples of (+)serum and (-)serum obtained in Example 2(2) was added onto NP20 chip at 1 µg per spot, dried and washed with water. After the addition of 5 mM DTT (in 25 mM NH₄HCO₃, pH 8) (5 µl), the chip was subjected to reduction treatment at 70°C. 5 ng of trypsin (in 25 mM NH₄HCO₃, pH 8) was added and the digestion was performed at 37°C for 2 hr. 20% CHCA (0.5 µl) was added twice, and mass spectrometry was performed using SELDI and PCI-Qstar, respectively. As a result, comparing (+)serum sample and (-)serum sample, plural (+)serum-specific peaks were confirmed (SELDI: Fig. 3, PCI-Qstar: Fig. 4). Peptide mass fingerprinting was performed by ProFound search (http://prowl.rockefeller.edu/profound_bin/WebProFound.exe) for these (+)serum-specific peaks. As a result, azurocidin was hit with a high probability of 95% or more. Of the (+)serum-specific peaks in peptide mass fingerprinting, the peaks predicted to be derived from azurocidin were those of M/Z 1181.15, 1505.79, 2861.40 and 3057.43 (corresponding to the fragments consisting of the amino acid sequence of the amino acid numbers 24-34, 11-23, 35-61 and 167-198 of the amino acid sequence shown in SEQ ID NO:2, respectively).

### (3) MS/MS analysis

Among the above-mentioned peaks predicted to be derived from azurocidin, the peak of M/Z 1505.7896 was subjected to MS/MS analysis using PCI-Qstar, the obtained peak was searched by MS-Tag (http://prospector.ucsf.edu/ucsfhtml4.0/mstag.htm). As a result, the peak was identified to correspond to the fragment from position 37 to position 49 of prepro azurocidin (the amino acid sequence of the amino acid numbers 11-23 of the amino acid sequence shown in SEQ ID NO:2). The search for a partial amino acid sequence of azurocidin was performed with the conditions: molecular weight 5,813, isoelectric point 8.5 or more, forming an S-S bond. As a result, it has been revealed that the fragment from position 36 to position 88 of prepro azurocidin (the amino acid sequence of the amino acid numbers 10-62 of the amino acid sequence shown in SEQ ID NO:2) has molecular weight of 5825.69 predicted from the amino acid sequence, predicted pI value of 10.66, and is predicted to form an S-S bond between the cysteine residue at the position 52 and the cysteine residue at the position 68. Consequently, this azurocidin-derived peptide (AZU(36-88)) was identified as a bone metastasis marker. Example 4 Confirmation of heparin bindability

Since azurocidin is a heparin-binding protein and has two heparin-binding sequence motifs in the molecule (the amino acid sequence of the amino acid numbers 5-10 and 61-65 of the amino acid sequence shown in SEQ ID NO:2, respectively), the adsorption of the objective peak to a heparin column was examined. An undenatured (+)serum sample was 10-fold diluted with column binding buffer (150 mM NaCl, 20 mM Tris-hydrochloric acid (pH 7.5)), and added to a heparin column (Heparin HyperD (registered trade mark) M; manufactured by BioSepra) equilibrated with the same buffer. The non-adsorbed fraction and the adsorbed fraction eluted with 2 M NaCl (pH 7.5) were sorted. Each fraction was concentrated, desalted and denatured, and the objective peak was detected therein using a cation exchange chip (pH 6.0) according to the method described in Example 1. As a result, the object peak markedly decreased in the non-adsorbed fraction, and the objective peak was detected in the fraction eluted form the column (Fig. 5).

### Industrial Applicability

Since the serum level of the peptide marker for bone metastasis of the present invention begins to increase at a certain time earlier than the exteriorization of the symptoms of bone metastasis, the marker makes it possible to detect and prevent bone metastasis earlier than diagnostic imaging currently utilized. Furthermore, since the analysis can be performed with a small amount of patient serum, the burden on the patient is less. Therefore, the peptide marker is extremely useful for application to a rapid, simple and economical diagnosis method of bone metastasis, as well as a reagent, a kit and the like therefor.

While this invention has been described with an emphasis upon preferred embodiments, it will be obvious to those of ordinary skill in the art that variations of the preferred embodiments may be used and that it is intended that the invention may be practiced otherwise than as specifically described herein. Accordingly, this invention includes all modifications encompassed within the spirit and scope of the invention as defined by the following claims.

This application is based on a patent application Nos. 2004-282822 and 2005-231193 filed in Japan, the contents of which are incorporated in full herein by this reference. In addition, all of the references cited herein, including patents, patent applications, and publications, are hereby incorporated in their entireties by reference.

## Claims

1. A peptide having the following properties:
(a) increased expression in the serum of a cancer patient over time from before the onset of bone metastasis to after the onset thereof;
(b) adsorbing to a weak cation exchanger under the condition of pH 6;
(c) apparent molecular weight by mass analysis: about 5,800.

2. A peptide consisting of the same or substantially the same amino acid sequence as the amino acid sequence of amino acid numbers 10-62 of the amino acid sequence shown in SEQ ID NO:2.

3. An antibody against the peptide of claim 1 or 2.

4. A diagnosis method of bone metastasis in a cancer patient, which comprises detecting the expression of the peptide of claim 1 or 2 in a biological sample derived from the patient.

5. The method of claim 4, wherein the biological sample is plasma or serum.

6. The method of claim 4 or 5, which comprises bringing the biological sample into contact with a cation exchanger, subjecting the component adsorbed to the cation exchanger in the sample to a mass spectrometry, and detecting a peptide having the molecular weight of about 5,800.

7. The method of claim 4 or 5, which comprises detecting the peptide of claim 1 or 2 using the antibody of claim 3.

8. The method of any one of claims 4 - 7, which comprises obtaining a biological sample from a cancer patient in time series, and assaying the time course expression of the peptide of claim 1 or 2 in the sample.


SEQUENCE LISTING

<110>  Kurume University

<120>  Novel Marker Peptide for Bone Metastasis and Method for
       Diagnosing Bone Metastasis Using Same

<130>  09820

<150>  JP 2004-282822
<151>  2004-09-28

<150>  JP 2005-231193
<151>  2004-08-09

<160>  2

<170>  PatentIn version 3.2

<210>  1
<211>  753
<212>  RNA
<213>  Homo sapiens


<220>
<221>  CDS
<222>  (1)..(753)

<220>
<221>  sig_peptide
<222>  (1)..(78)

<220>
<221>  mat_peptide
<222>  (79)..(744)

<400>  1
aug acc cgg cug aca guc cug gcc cug cug gcu ggu cug cug gcg ucc       48
Met Thr Arg Leu Thr Val Leu Ala Leu Leu Ala Gly Leu Leu Ala Ser
    -25                 -20                 -15

ucg agg gcc ggc ucc agc ccc cuu uug gac auc guu ggc ggc cgg aag       96
Ser Arg Ala Gly Ser Ser Pro Leu Leu Asp Ile Val Gly Gly Arg Lys
-10                 -5                  -1  1               5

gcg agg ccc cgc cag uuc ccg uuc cug gcc ucc auu cag aau caa ggc      144
Ala Arg Pro Arg Gln Phe Pro Phe Leu Ala Ser Ile Gln Asn Gln Gly
            10                  15                  20

agg cac uuc ugc ggg ggu gcc cug auc cau gcc cgc uuc gug aug acc      192
Arg His Phe Cys Gly Gly Ala Leu Ile His Ala Arg Phe Val Met Thr
        25                  30                  35

gcg gcc agc ugc uuc caa agc cag aac ccy ggg guu agc acc gug gug      240
Ala Ala Ser Cys Phe Gln Ser Gln Asn Pro Gly Val Ser Thr Val Val
    40                  45                  50

cug ggu gcc uau gac cug agg cgg cgg gag agg cag ucc cgc cag acg      288
Leu Gly Ala Tyr Asp Leu Arg Arg Arg Glu Arg Gln Ser Arg Gln Thr
55                  60                  65                  70

uuu ucc auc agc agc aug agc gag aau ggc uac gac ccc cag cag aac      336

Phe Ser Ile Ser Ser Met Ser Glu Asn Gly Tyr Asp Pro Gln Gln Asn
75 80 85

cug aac gac cug aug cug cuu cag cug gac cgu gag gcc aac cuc acc 384
Leu Asn Asp Leu Met Leu Leu Gln Leu Asp Arg Glu Ala Asn Leu Thr
90 95 100

agc agc gug acg aua cug cca cug ccu cug cag aac gcc acg gug gaa 432
Ser Ser Val Thr Ile Leu Pro Leu Pro Leu Gln Asn Ala Thr Val Glu
105 110 115

gcc ggc acc aga ugc cag gug gcc ggc ugg ggg agc cag cgc agu ggg 480
Ala Gly Thr Arg Cys Gln Val Ala Gly Trp Gly Ser Gln Arg Ser Gly
120 125 130

ggg cgu cuc ucc cgu uuu ccc agg uuy guc aac gug acu gug acc ccc 528
Gly Arg Leu Ser Arg Phe Pro Arg Phe Val Asn Val Thr Val Thr Pro
135 140 145 150

gag gac cag ugu cgc ccc aac aac gug ugc acc ggu gug cuc acc cgc 576
Glu Asp Gln Cys Arg Pro Asn Asn Val Cys Thr Gly Val Leu Thr Arg
155 160 165

cgc ggu ggc auc ugc aau ggg gac ggg ggc acc ccc cuc guc ugc gag 624
Arg Gly Gly Ile Cys Asn Gly Asp Gly Gly Thr Pro Leu Val Cys Glu
170 175 180

ggc cug gcc cac ggc gug gcc ucc uuu ucc cug ggg ccc ugu ggc cga 672
Gly Leu Ala His Gly Val Ala Ser Phe Ser Leu Gly Pro Cys Gly Arg
185 190 195

ggc ccu gac uuc uuc acc cga gug gcg cuc uuc cga gac ugg auc gau 720
Gly Pro Asp Phe Phe Thr Arg Val Ala Leu Phe Arg Asp Trp Ile Asp
200 205 210

ggu guu cuc aac aac ccg gga ccg ggg cca gcc 753
Gly Val Leu Asn Asn Pro Gly Pro Gly Pro Ala
215 220 225


<210> 2
<211> 251
<212> PRT
<213> Homo sapiens

<220>
<221> misc_feature
<222> (48)..(48)
<223> The 'Xaa' at location 48 stands for Pro.

<400> 2

Met Thr Arg Leu Thr Val Leu Ala Leu Leu Ala Gly Leu Leu Ala Ser
-25 -20 -15


Ser Arg Ala Gly Ser Ser Pro Leu Leu Asp Ile Val Gly Gly Arg Lys
-10 -5 -1 1 5


Ala Arg Pro Arg Gln Phe Pro Phe Leu Ala Ser Ile Gln Asn Gln Gly
10 15 20

Arg His Phe Cys Gly Gly Ala Leu Ile His Ala Arg Phe Val Met Thr
        25                  30                  35

Ala Ala Ser Cys Phe Gln Ser Gln Asn Xaa Gly Val Ser Thr Val Val
    40                  45                  50

Leu Gly Ala Tyr Asp Leu Arg Arg Arg Glu Arg Gln Ser Arg Gln Thr
55                  60                  65                  70

Phe Ser Ile Ser Ser Met Ser Glu Asn Gly Tyr Asp Pro Gln Gln Asn
                75                  80                  85

Leu Asn Asp Leu Met Leu Leu Gln Leu Asp Arg Glu Ala Asn Leu Thr
            90                  95                  100

Ser Ser Val Thr Ile Leu Pro Leu Pro Leu Gln Asn Ala Thr Val Glu
        105                 110                 115

Ala Gly Thr Arg Cys Gln Val Ala Gly Trp Gly Ser Gln Arg Ser Gly
    120                 125                 130

Gly Arg Leu Ser Arg Phe Pro Arg Phe Val Asn Val Thr Val Thr Pro
135                 140                 145                 150

Glu Asp Gln Cys Arg Pro Asn Asn Val Cys Thr Gly Val Leu Thr Arg
                155                 160                 165

Arg Gly Gly Ile Cys Asn Gly Asp Gly Gly Thr Pro Leu Val Cys Glu
            170                 175                 180

Gly Leu Ala His Gly Val Ala Ser Phe Ser Leu Gly Pro Cys Gly Arg
        185                 190                 195

Gly Pro Asp Phe Phe Thr Arg Val Ala Leu Phe Arg Asp Trp Ile Asp
    200                 205                 210

Gly Val Leu Asn Asn Pro Gly Pro Gly Pro Ala
215                 220                 225